# EUROPEAN PATENT SPECIFICATION

(11) **EP 0 513 224 B1**
(45) Date of publication and mention of the grant of the patent: **23.10.1996**
(21) Application number: 91904670.6
(22) Date of filing: 31.01.1991
(51) Int. Cl.: A61M 37/00, A61M 25/01

(54) **CATHETER STEERING MECHANISM**
LENKUNGSMECHANISMUS FÜR KATHETER
MECANISME DE GUIDAGE DE CATHETERS

(30) Priority: 02.02.1990 US 473667
(43) Date of publication of application: 19.11.1992
(73) Proprietor: EP TECHNOLOGIES, INC., Sunnyvale, California 94086 (US)
(72) Inventor: LUNDQUIST, Ingemar, Pebble Beach, CA 93953 (US); THOMPSON, Russell, Blake, San Leandro, CA 94578 (US)
(74) Representative: Adams, William Gordon
(86) International application number: US9100662
(87) International publication number: WO9111213

(56) References cited:
- US-A- 3 470 876
- US-A- 3 547 103
- US-A- 3 625 200
- US-A- 4 586 923
- US-A- 4 719 924
- US-A- 4 921 482
- US-A- 4 934 340
- US-A- 4 944 727
- Surgery, Vol. 27, No. 6, June 1950, pp 817-821, (SMITH et al.) "Preliminary report on a new method of intestinal intubation with the aid of a flexible stylet with controllable tip".

## Description

This invention relates to a steering mechanism for use with medical catheters or other devices which need to be positioned within difficult locations.

In many medical procedures, it is necessary to position a catheter at a location within a patient's body. A typical emplacement for the distal end of a catheter might be within a ventricle of the heart, by way of the femoral vein. In so passing a catheter through the femoral vein, it is necessary to avoid obstructions, vessel junctions and the like, and to make sharp turns to position the distal end of the catheter within the ventricle. Other medical procedures involve similar difficulties in placing a catheter.

In conventional catheters used today, the tip of the catheter may be bent, or may include a stylet which is bent, such that a semipermanent curve is given to the distal end of the catheter so that a physician may guide the distal end thereof towards the treatment location. A disadvantage with this type of apparatus is that the curvature of the bend is not adjustable while the catheter is in the body, and any change in the curvature requires the physician to remove the catheter and reshape the distal end.

There is a need for a steering mechanism for catheters and other devices wherein the distal end of the device can be manipulated at will from a location outside the patient's body or outside the apparatus in which the device is placed. Accordingly, it is an object of the present invention to provide such a steering mechanism, and in particular to provide such a mechanism which provides a wide range of steerability.

In an article "Preliminary report on a New Method of Intestinal Intubation with the Aid of a Flexible Stylet with Controllable Tip", in "Surgery", Vol. 27, No. 6, June 1950, pp 817-21, Smith et al disclose apparatus comprising a long heavily constructed coil spring body from which extends a flexible column of coiled flat wire carrying at its remote end a controllable tip. Fine wires pass through the body and column to the point of the tip, which are tensioned by a handle attached to the body to cause the tip to flex in either of two opposed directions. Although the tip of this apparatus comprises a flat spring, the latter is completely enclosed within a coiled spring.

The present invention provides a steering mechanism, including:
a steering shaft having a proximal end and a distal end and a lumen therethrough;
a coiled spring mounted within said steering shaft;
a lead spring positioned within said distal end;
at least one steering wire extending through said lumen and having a distal end attached to a first portion of said lead spring, for bending the lead spring towards said steering wire when tension is applied to the steering wire, thus bending the distal end of the steering shaft; and
a controller positioned at the proximal end of the steering shaft and attached to the proximal end of said at least one steering wire, for placing tension on said steering wire;
characterised in that the lead spring is secured at the distal end of said coiled spring against longitudinal movement relative to said distal end, said first portion of said lead spring extending beyond said coiled spring.

In use, the steering shaft is inserted into a lumen of a catheter, and the controller is manipulated to turn the distal end of the catheter.

For other features of the invention, reference should be made to the appended claims, and also to the embodiments, which will now be described with reference to the accompanying drawings, in which:
Figure 1 shows a steering mechanism according to the invention.
Figure 2 is an enlarged perspective view of a portion of the mechanism of Figure 1.
Figure 3 is an enlarged perspective view similar to Figure 2, showing a different position for the mechanism.
Figure 4 is a sectional view of the mechanism of Figure 1 in yet another position.
Figure 5 is a sectional view taken along line 5-5 of Figure 4.
Figure 6 is a sectional view taken along line 6-6 of Figure 1.
Figure 7 is a perspective view of an alternative embodiment of the invention.
Figure 8 is a sectional view of a distal end of the embodiment of Figure 7.
Figure 9 is a sectional view taken along line 9-9 of Figure 7.
Figure 10 is a sectional view taken along line 10-10 of Figure 9.
Figure 11 is a perspective view of another embodiment of the invention.
Figure 12 is a sectional view taken along line 12-12 of Figure 11.
Figure 13 is a sectional view taken along line 13-13 of Figure 12.
Figure 14 is a perspective view of yet another embodiment of the invention.
Figure 15 is a sectional view taken along line 15-15 of Figure 14.
Figure 16 is a perspective view, partially cut away, of another embodiment of the invention.
Figure 17A is a top view, partly cut away, of the embodiment of Figure 16.
Figure 17B is a sectional view taken along line 17B-17B of Figure 17A.
Figure 18 is a perspective view of another embodiment of the invention.
Figure 19 is a top view of the embodiment of Figure 18.
Figure 20 is a perspective view of another embodiment of the invention.
Figure 21 is a sectional view of the distal end of the embodiment of Figure 20.
Figure 22 is a sectional view like that of Figure 21, in a straightened position.
Figure 23 is a top view, partially cut away, of the embodiment of Figure 20.
Figure 24 is a sectional view taken along line 24-24 of Figure 23.
Figure 25 is a sectional view of an alternative embodiment to the distal end of a steering mechanism of the invention.
Figure 26 is a sectional view taken along line 26-26 of Figure 25.
Figure 27 is a sectional view taken along line 27-27 of Figure 25.
Figure 28 is a sectional view taken along line 28=28 of Figure 25.
Figure 29 is an exploded view showing detail of a portion of the invention.
Figure 30 is a sectional view of a portion of the steering mechanism of the invention.
Figure 31 is a sectional view taken along line 31-31 of Figure 30.
Figure 32 is a sectional view taken along line 32-32 of Figure 30.
Figures 33 and 34 are sectional views of an alternative embodiment of the invention.
Figure 35 shows the embodiment of Figure 1 in use with a conventional medical catheter.
Figure 36 shows an alternative embodiment to the distal tip of the invention.

### Description of the Preferred Embodiments

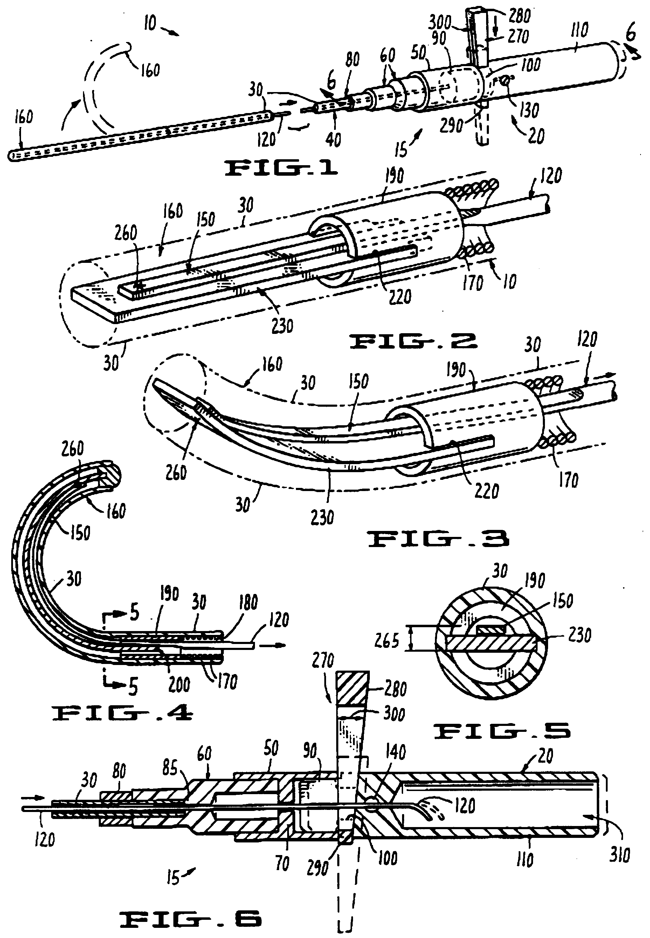

Figure 1 shows a steering mechanism 10 including a controller 15 with a handle 20. A flexible steering shaft 30 is attached at its proximal end 40 to the controller 15. The shaft 30 is formed from any conventional material which is flexible, slightly elastic to tolerate bending without permanent deformation, and biocompatible for applications in the medical field. Preferably, the shaft 30 is formed from TEFLON (tetrafluoroethylene) for minimizing friction with other elements of the apparatus.

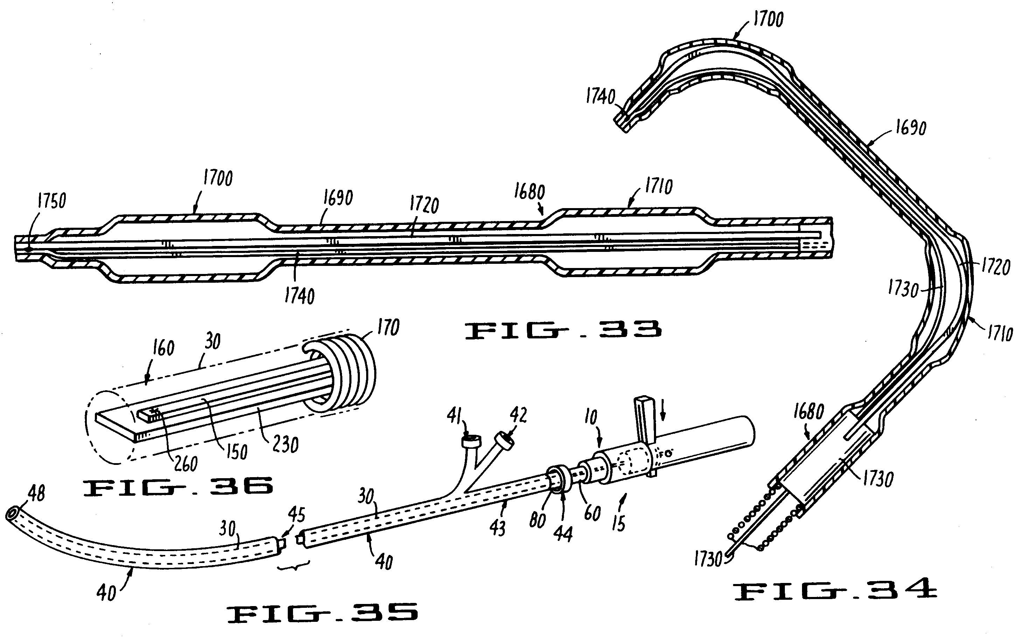

The mechanism 10 may be used to steer and position many different types of apparatus, and the embodiment shown in Figure 1 is adapted for use with medical catheters. As shown in Figure 35, the steering shaft 30 is positioned within a lumen 45 of a catheter 40, the distal tip 48 of the catheter 40 is maneuvered to a desired position within a patient's body, and the steering mechanism may then be removed so that the lumen 45 may then be used by the physician for introduction of treatment drugs or the like to the patient. The catheter may include also conventional ports for this purpose, such as ports 41 and 42 shown in Figure 35 which communicate with one or more additional lumens (not separately shown) within the catheter 40.

The catheter 40 is any standard catheter, such as an angioplasty catheter (for laser angioplasty, balloon angioplasty, or other treatments), a catheter with pacing and/or sensing probes, or indeed any flexible or steerable device which one may wish to introduce into a place which is difficult to reach. Such devices need not be confined to the medical field. Other important applications include the introduction of tools (for inspection, adjustment, or repair) into engines or other machines, and the manipulation of objects in any type of conduit. For inspection purposes, fiber optics may be carried by the catheter 40 or other device. In machine repair and adjustment applications, any convention tool may be attached to the distal end of the steering shaft 30, such as drill bits, clamps, wrenches, grinders, and so on, and these tools may be remotely operated from the controller 15. Thus, although the present embodiments of the invention are described primarily in terms of use with a catheter, the invention has many other applications.

In the course of the following discussion, it will be understood that the manipulation, steering or positioning of the shaft 30 (or other shafts of the alternative embodiments) results in concomitant manipulation, steering or positioning of the catheter or other device with which the steering mechanism is used. Any of the following embodiments may be used with the catheter 40 shown in Figure 35 or other steerable device.

The steering mechanism 10 includes a first sleeve 50, as shown in Figures 1 and 6, adjacent which is positioned a second sleeve 60. The sleeve 50 includes a stop 70 at its interior, as shown in Figure 6, against which the second sleeve 60 abuts. The second sleeve 60 has an outer diameter closely matching the inner diameter of the first sleeve 50.

A third sleeve 80 is positioned within a forward portion of the second sleeve 60, and has an outer diameter closely matching the inner diameter of the forward portion. The third sleeve 80 has an inner diameter closely matching the outer diameter of the steering shaft 30. The sleeves 50, 60 and 80 are firmly fixed so that they do not move relative to one another. As shown in Figure 6, the steering shaft 30 is seated against a shoulder 85 of the sleeve 60. The sleeve 60 acts as a cable or wire retainer for the steering wire 120.

Alternatively, for ease of manufacture the sleeves 50, 60 and 80 may be of a unitary design.

As shown in Figure 35, the catheter 40 includes a proximal end 43 which fits tightly against the sleeves 60 and 80 when the steering shaft 30 is inserted all the way into the lumen 45 and is seated against shoulder 85 shown in Figure 6. The end 43 may be attached to the controller 15 by means of a standard Luer lock 44.

The handle 20 shown in Figures 1 and 6 is generally cylindrical in shape and includes a forward section 90 (shown in dotted fashion in Figure 1), a middle section 100, and a rear section 110. The forward section 90 has an outer diameter closely matching the inner diameter of the first sleeve 50, and is positioned within the sleeve 50 in a slidable fashion. The middle section 100 has a reduced outer diameter, and the rear section 110 has an outer diameter which is chosen for a comfortable grasp by the hand of the operator.

The sleeves 50, 60, and 80 and the three sections of the handle 20 all include central bores which are coaxially aligned for receiving a steering wire 120, as shown in Figure 6. One end of the wire 120 is clamped in position relative to the handle 20 by means of a screw 130, shown in Figure 1, which is threaded into a bore 140, shown in Figure 6.

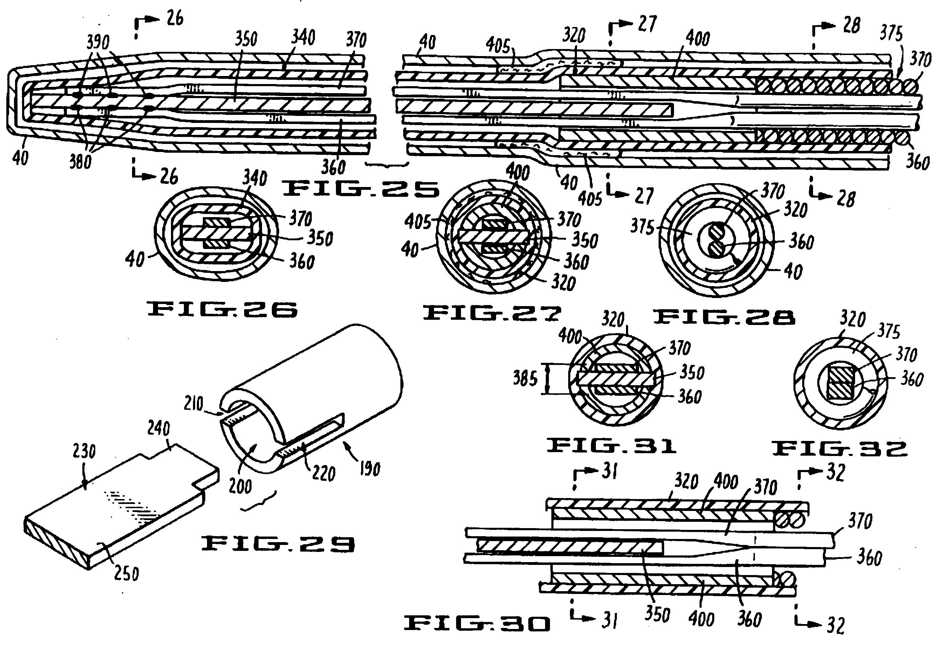

The steering wire 120 includes a distal end 150 which is flattened in the preferred embodiment such that it has a rectangular cross section, and extends into a distal end 160 of the shaft 30. A coiled spring or sleeve 170 defines a central lumen 180 within the steering shaft 30, in which lumen the steering wire 120 is positioned. A ferrule 190 is positioned within the shaft 30 immediately distal of, and abutting, the coiled spring 170. The ferrule 190 includes a central lumen 200, and two slots 210 and 220 (as shown in Figure 29) which are disposed radially opposite one another, and together form a single transverse slot.

A flat, flexible lead spring 230 is positioned within the slots 210 and 220. The lead spring 230 includes a tongue 240 on its proximal end, which has a width closely matching the inner diameter of the ferrule 190. The lead spring 230 also includes a broader portion 250 with an outer diameter closely matching the outer diameter of the ferrule 190. Thus, when the lead spring 230 is positioned as shown in Figures 2 and 3, the tongue 240 (Figure 29) is within the ferrule 190, and the proximal end of the broader portion 250 abuts the bottom ends of the slots 210 and 220. With the ferrule 190 and lead spring 230 in the position shown in Figure 1, the distal end 150 of the steering wire 120 is welded or otherwise attached to the lead spring 230, such as at weld 260.

As shown in Figure 5, the ferrule 190 and the shaft 30 have inner diameters which are substantially greater than the combined thickness 265 of the distal end 150 of the steering wire 120 and the lead spring 230.

In an alternative embodiment, shown in Figure 36, the ferrule 190 may be omitted, in which case the lead spring 230 is inserted into the coiled spring 170 in much the same fashion as into the ferrule 190, with the same dimensions applying to this embodiment. The lead spring 230 may be attached to the coiled spring 170 by adhesive, solder, welds, or the like.

Referring now to Figures 1 and 6, the middle section 100 of the handle 20 includes a transverse slot therethrough, into which a control wedge 270 is positioned. The wedge 270 includes a larger end 280 tapering towards a smaller end 290. The wedge 270 also includes a slot 300 extending most of its length, as shown in Figures 1 and 6. The wedge 270 is positioned such that the wire 120 passes through the slot 300, as shown in Figure 6, in which position the slot 300 is generally parallel to the longitudinal axis of the handle 20, and also is parallel to the proximal end of the wire 120. The ends 280 and 290, which form the ends of the slot 300, prevent the wedge 270 from accidental removal.

When the wedge 270 is in the upper position shown in Figures 1 and 6, the distal end 160 of the shaft 30 has the configuration shown in Figure 2. However, when the wedge 270 is pushed downward to the position shown in dotted fashion in Figures 1 and 6, this forces the first sleeve 50 forwardly relative to the handle 20, by increasing the distance therebetween. Alternatively speaking, the handle 20 is forced rearwardly relative to the sleeve 50, into the position shown in dotted fashion (at the extreme right end) in Figures 1 and 6. The proximal end of the wire 120, being affixed by the screw 130, is similarly moved towards the right from the point of view of Figure 6, to the position shown in dotted fashion.

The wire 120 is thus pulled toward the right from the point of view of Figures 1-4 and 6, and thereby pulls the lead spring 230 at the weld 260, as shown in Figure 3. This bends the lead spring 230 in the direction of the distal end 150 of the wire 120, thus bending the distal end 160 of the shaft 30, as shown in Figures 2 through 4.

It will be appreciated that, because of the relatively large inner diameters of the ferrule 190 and the shaft 30, lateral torque or leverage may be exerted by the distal end 150 on the lead spring 230, in order to effect bending of the distal end 160 of the shaft 30. Thus, by providing enough radial play to the distal end 150 of the wire 120 and the lead spring 230, sufficient torque for steering the distal end 160 may be assured. The amount of radial play may be determined empirically for a given application, but in most cases a minimum inner diameter of the lead spring 230 and the shaft 30 of approximately two to three times the combined thickness 165 shown in Figure 5 will be sufficient to provide the necessary torque.

The materials for the wedge 270, the handle 20 and the sleeve 50 are chosen to provide sufficient friction, such that the wedge 270 maintains its position wherever it is located by the operator, so that the distal end 160 can be fixed in a variety of positions simply by repositioning the wedge 270. The wire 120 may be tightened by loosening the screw 130, pulling the wire 120 tight, and then retightening the screw 130, which results in a change in the frictional fit of the wedge 270. Access to the proximal end of the wire 120 may be had through an opening 310 in the rear section of the handle 130.

In the preferred embodiment, the lead spring 230 is affixed to the ferrule 190, and the ferrule 190 is in turn affixed to the coiled spring 170. This may be done by adhesive, welds or the like, and is especially useful in conjunction with a catheter which is to be emplaced permanently or for a long period of time within a patient. The ferrule may alternatively simply be abutted against the coiled spring 170, in which case the distal end 160 of the steering shaft is preferably formed from a heat shrink or other tightly fitting material to maintain the ferrule firmly in place.

In use, a physician will insert the catheter 40 into a patient, such as into the femoral vein, and will insert the shaft 30 into the lumen 45. By manipulating the distal end 160 as necessary and pushing on the handle 20, the physician can negotiate a path to the treatment site, such as the heart. The physician may, by operating the control wedge 270, steer past any junction, turn or obstacle which may be presented to the catheter simply by adjusting the control wedge such that the distal end 160 of the shaft 30, and hence also the distal end 48 of the catheter 40, takes on the necessary shape. Once a given obstacle or turn is negotiated by the end 160, it can then again be straightened out by operation of the wedge 270, or may be changed to a different shape as necessary. If, for instance, the physician encounters a turn of the vein towards the right, the wedge 270 is pushed downward as shown in Figure 1, such that the distal end 160 takes on the position shown in dotted fashion. If the vein then takes a turn to the left, the physician straightens out the distal end 160, rotates the handle 20 such that the distal end 160 rotates the necessary amount (such as 180°), and then again operates the wedge 270 to manipulate the distal end 160 around the left turn. In this way, complete control over steering and emplacement of the catheter 40 is achieved.

Figures 7-10 show an alternative embodiment, wherein left and right turns may be negotiated without rotation of the apparatus. The steering mechanism 315 shown in Figure 7 includes a flexible steering shaft 320 having a proximal end 330 and a distal end 340 and may be identical to the shaft 30 shown in Figure 1. As shown in Figure 8, carried within the distal end 340 are a lead spring 350 and two steering wires 360 and 370. The steering wires 360 and 370 are preferably rounded towards their proximal ends, and flattened towards their distal ends, as with the steering wire 120 shown in Figure 1. The steering wires 360 and 370 are attached to the distal end of the lead string 350 by solder, adhesive, spot welds or the like, such as at welds 380 and 390. For minimizing friction, the steering wires 360 and 370 (and the steering wires of any of the embodiments described herein) are preferably coated with TEFLON.

The lead spring 350, which may be identical to the lead spring 230 shown in Figure 2, is seated in a ferrule 400, which is shown in Figure 8 and may be identical to the ferrule 190 shown in Figure 2. Thus, the configuration of the distal end 340 of the shaft 320 shown in Figure 8 is essentially identical to the distal end 160 of the shaft 30 shown in Figure 2, except that an additional steering wire is attached to the lead spring 350. The mechanism 315 may be used in the same applications as the mechanism 10.

Figures 25-28 show further detail of the distal tip of a steering shaft of the embodiment of Figures 7-10, with Figures 26, 27 and 28 showing cross-sectional views at the regions indicated in Figure 25 along the length of the shaft 320. The numerals appearing in Figures 25-28 are the same as those in Figures 7 and 8, but the details of these figures may be employed in any of the other embodiments described herein which utilize two steering wires.

In Figures 25-28, the shaft 320 is shown as used in conjunction with the catheter 40 shown in Figure 35. Preferably, the catheter 40 includes a standard braided or other torquable material, and the shaft 320 is adhered to the interior of the catheter 40 by adhesive means such as glue 405 shown in Figures 25 and 27. With this configuration, torque on the proximal end of the catheter 40 will be transmitted to its distal end, and via the glue 405 will be transmitted to the shaft 320, thus allowing for turning of the distal end 340. Alternatively, and especially in embodiments wherein no catheter is used, the shaft 320 may be made of a torquable material.

Distal end 340 of the shaft 320 receives the lead spring 350 and the steering wires 360 and 370, as shown in Figure 25. The rounded or circular proximal ends of the steering wires 360 and 370 are positioned within a coiled sleeve 375, as shown in Figure 28. The coiled sleeve 375 may be a conventional metal coil such as those used in medical catheters to provide longitudinal stiffness but lateral flexibility. The steering wires 360 and 370 are flattened into a rectangular shape in the region near the lead spring 350.

In the preferred embodiment, the steering wires 360 and 370 are attached at several places to the lead spring 350, such as at the three spot welds 380 and the three spot welds 390, respectively, shown in Figure 25. The distal end 340 may be a heat-shrink material which, once the elements of the steering mechanism are assembled, is heated to reduce its diameter relative to the main body of the shaft 320, thus tapering the tip in the region of the spot welds 380 and 390, as shown in Figure 25. When spot welds are used to attach the steering wires to the lead spring, the use of heat-shrink is particularly advantageous in applying pressure to the welded region to prevent separation. When solder is used, this is not as important a consideration.

Figures 30-32 show an alternative embodiment of the distal end of the invention, wherein the proximal portions of the steering wires 360 and 370 are of non-circular cross section, such as the rectangular cross section shown in this embodiment. The steering wires 360 and 370 may be further flattened, as shown in Figure 31, in the region of the lead spring 350, thus providing greater clearance between the combined thickness 385 of the steering wires 360 and 370 and the lead spring 350, on the one hand, and the inner diameters of the ferrule 400 and the shaft 320, on the other hand. As discussed above relative to Figures 1-5 (and as shown in Figure 5), this clearance allows greater torque to be placed upon the distal tip of the steering shaft when the physician turns the distal tip by placing tension on the steering wires.

Referring again to Figures 7-10, it will be seen that the configuration shown in Figures 25-28 (or 30-32) allows either left or right turns to be effected by pulling on the wire 360 or the wire 370, as shown in dotted fashion for the distal end 340 in Figure 7. Figure 8 shows the configuration of the distal end 340 of the shaft 320 for a right turn, wherein rearward tension is placed on the steering wire 370 (represented by the right-facing arrow), and a forward force is simultaneously applied to the steering wire 360 (as represented by the left-facing arrow). The forward force to the wire 360 occurs both because of the push given to the wire 360 by the clockwise rotation of the spindle 450 (which is described in greater detail below), and because of the pull exerted by the wire 370 via the welds 390, the lead spring 350, and the welds 380. When the spindle 450 is rotated counterclockwise, force is placed on the steering wires 360 and 370 in directions opposite to the arrows shown in Figure 8, thus bending the distal end 340 to the left, as shown in dotted fashion.

In order to provide such tension to the steering wires, a controller 410 is provided, as shown in Figures 7, 9 and 10. The controller 410 includes a housing 420 which receives the proximal end 330 of the shaft 320. Attached to the housing 420 is a coupling 430 which has a central bore 440 (shown in dotted fashion in Figure 9) which is substantially coaxial with the lumen of the shaft 320.

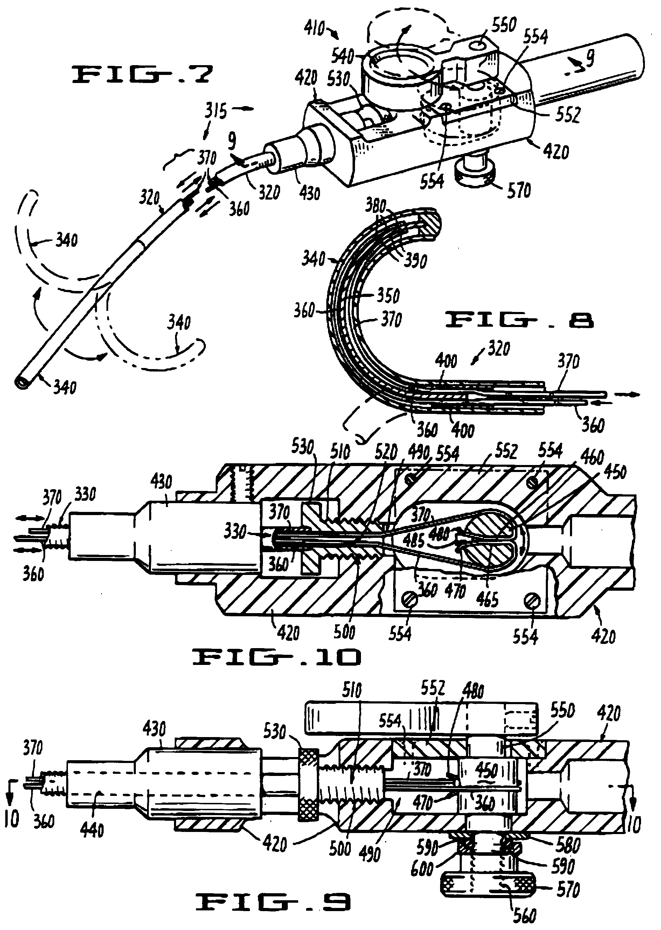

The guide wires 360 and 370 pass through the bore 440, and are attached as shown in Figures 9 and 10 to a spindle 450. The preferred means of attachment is by insertion through a bore 460 in the spindle 450, and the proximal tips 470 and 480 of the steering wires 360 and 370, respectively, are bent to prevent retraction through the bore 460. The wires are chosen to be stiff enough that they will retain the bent shape at their proximal tips, and will not retract from the bore 460. Optionally, a taper pin 485 may also be used, being driven between the ends 470 and 480 into a frictional fit in a flared portion 465 of the bore 460, thus preventing the proximal tips 470 and 480 from accidental withdrawal.

The housing 420 includes a central passage 490, through which the steering wires 360 and 370 pass, and may include a threaded portion 500 in which an adjustable, threaded tension adjusting screw or bolt 510 is positioned. The tension bolt 510 includes a central bore 520, as shown in Figure 10, and a knurled knob 530 for adjustment by hand. As shown in Figure 7, the knob 530 may be accessed by the operator of the controller 410 at any time, without disassembling the steering mechanism. The knob 530 is turned to adjust tension on the steering wires 360 and 370, and to adjust the amount of play in the lead spring 350, as described in greater detail below.

The spindle 450 is mounted on an axis which is preferably transverse to a longitudinal axis of the housing 420, and is rotatable by means of a steering handle 540, which is connected to the spindle 450 by a shaft 550. The spindle 450 and associated steering apparatus are affixed to the housing by means of a plate 552 which is mounted to the housing 420 by means of screws or bolts 554.

The shaft 550 includes a threaded bottom end 560, onto which a tightening knob 570 is threaded. A washer 580 is preferably positioned between the knob 570 and the housing 420, and a resilient O-ring 590 is positioned adjacent the washer 580. Between the O-ring 590 and the knob 570 is positioned another washer 600.

The washers 580 and 600 and the O-ring 590 are all coaxial with and surrounding the threaded portion 560 of the shaft 550. Thus, when the knob 570 is rotated such that it is tightened (i.e., moved closer to the housing 420), the increased friction between the knob 570, the washers 580 and 600, the O-ring 590 and the housing 420 inhibits the shaft 550 from turning, thereby maintaining the handle 540 in any position set by the operator of the steering mechanism. The resiliency of the O-ring 590 allows the operator to adjust the friction by means of the knob 570 such that the handle 540 may be relatively easily rotated by pushing with the fingers, and yet such that the distal end 340 will not spring back to the straightened shape shown in Figure 7 (which would cause the handle 542 spring back to the central position shown in Figure 7) when the operator releases the handle 540. Alternatively, the knob 570 may be loosened such that whenever the operator manipulates the handle 540, and then releases the handle, the distal end 340 is allowed to straighten out--which it will do because of the tension of one or the other of the steering wires, such as the tension due to the steering wire 360 in the position shown in Figure 8.

In order to increase the tension in the steering wires 360 and 370 and decrease the rotational play in the handle 540, the knob 530 is rotated such that it moves to the left from the point of view of Figure 10 (which will be a counterclockwise rotation for right-hand threads), thereby exerting force on the proximal end 330 of the coiled sleeve 375 (the force being towards the left in Figure 10). The sleeve 330 is longitudinally relatively rigid, and thus the force on its proximal end is transmitted to its distal end, where it is exerted in turn on the ferrule 400, and thence onto the lead spring 350. The force on the lead spring 350 is transmitted to the steering wires 360 and 370 via the welds 380 and 390. Thus, any longitudinal play in the system is reduced or eliminated by the counterclockwise rotation of the tension bolt 510, which results in greater responsiveness in the rotational control of the lead spring 350 by means of the steering handle 540.

Conversely, tension on the steering wires is reduced, and greater play is given to the steering function, by rotating the knob 530 such that it moves towards the right from the point of view of Figure 10.

The embodiment of Figures 7-10, therefore, allows the operator of the steering mechanism to manipulate the catheter into whatever position desired, accommodating both left and right turns through the passages encountered in the body. At most, the distal end 340 of the shaft 320 will need to be rotated 90° to accommodate all possible twists and turns that might be encountered in the body. The controller 410 may be manipulated entirely with one hand, including both operating the handle 540 to position the distal end 340 into the right and left turn configurations shown in dotted fashion in Figure 7, and also including axial rotations of the distal end 340 should those become necessary.

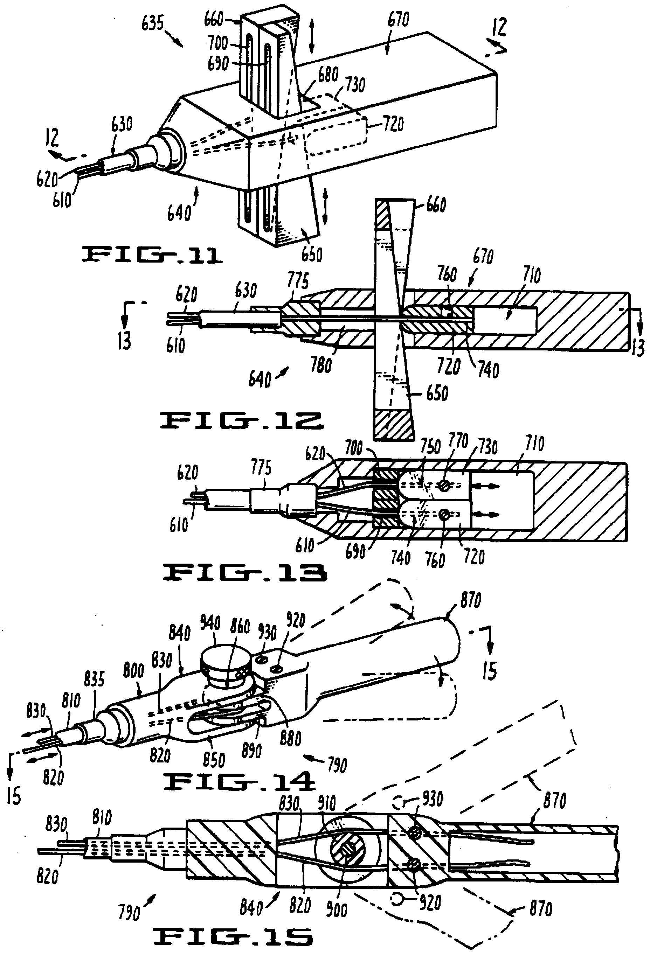

Figures 11-13 show an alternative embodiment of the invention comprising a steering mechanism 635 which incorporates two steering wires 610 and 620 which extend through a flexible steering shaft 630. The portion of the mechanism extending to the left from the point of view of Figure 11 may be identical to the embodiment shown in Figures 7 and 8. The steering mechanism 635 of Figures 11-13 includes a controller 640 which constitutes an alternative to the controller 410 shown in Figures 7, 9 and 10.

The controller 640 includes a left control wedge 650 and a right control wedge 660 mounted in a housing 670. The housing 670 includes an opening 680 in which the control wedges 650 and 660 are positioned as shown in Figures 11 and 12, with control wedge 650 being tapered upwardly, and control wedge 660 being tapered downwardly. The wedges 650 and 660 may be identical, except that they are carried in opposite directions in the housing 670.

Control wedge 650 includes a slot 690, and control wedge 660 includes a slot 700. These slots terminate at the upper and lower ends of the control wedges, as shown in Figure 11.

The housing 670 includes a cavity 710 in which a left control block 720 and a right control block 730 are positioned, such that they may slide to the left and right from the point of view of Figures 11-13 (as shown by the double arrows in Figure 13). The left control block 720 includes a bore 740, and the right control block 730 includes a bore 750, into which the extreme proximal ends of the steering wires 610 and 620 extend, as shown in Figure 13. Screws 760 and 770 are provided, as shown in Figures 12 and 13, and are threaded into the control blocks 720 and 730 for clamping down the proximal ends of the steering wires 610 and 620.

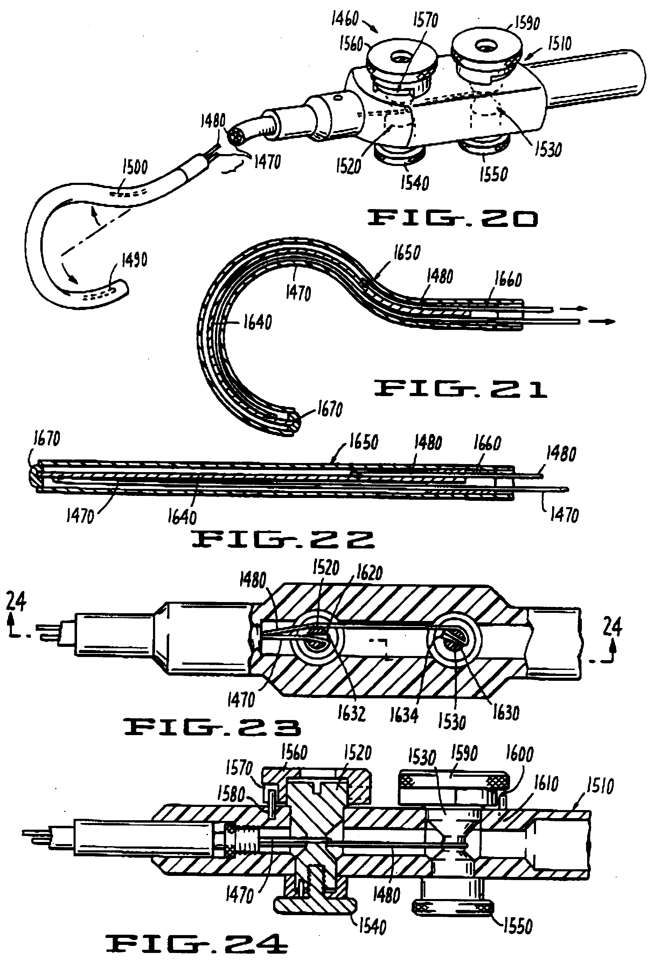

As shown in Figures 11-13, the steering wires 610 and 620 extend from the steering shaft 630 through a wire retainer 775 and through a bore 780 in the distal end of the housing 670, through the slots 690 and 700, and thence into the bores 740 and 750, where they are held in place by the screws 760 and 770. Thus, if left control block 720 is forced to the right from the point of view of Figure 13, then tension is placed on steering wire 610, thereby turning the distal tip of the steering shaft 630 (and also the distal tip of the catheter in which the shaft is positioned) to the left (as shown in dotted fashion in Figure 7). Likewise, if right control block 730 is forced to the right, tension is placed on the steering wire 620, forcing the distal tip of the steering shaft 630 (and the distal tip of the catheter) to the right. Again, only one hand is needed to operate the controller 640, and at most a 90° turn of the distal tip of the steering shaft 630 will be required to negotiate all twists and turns within the passages in the body of a patient. As will be discussed in further detail below, the controller 640 of Figures 11-13 has particular advantages when used with a distal tip of the configuration shown in Figures 21 and 22.

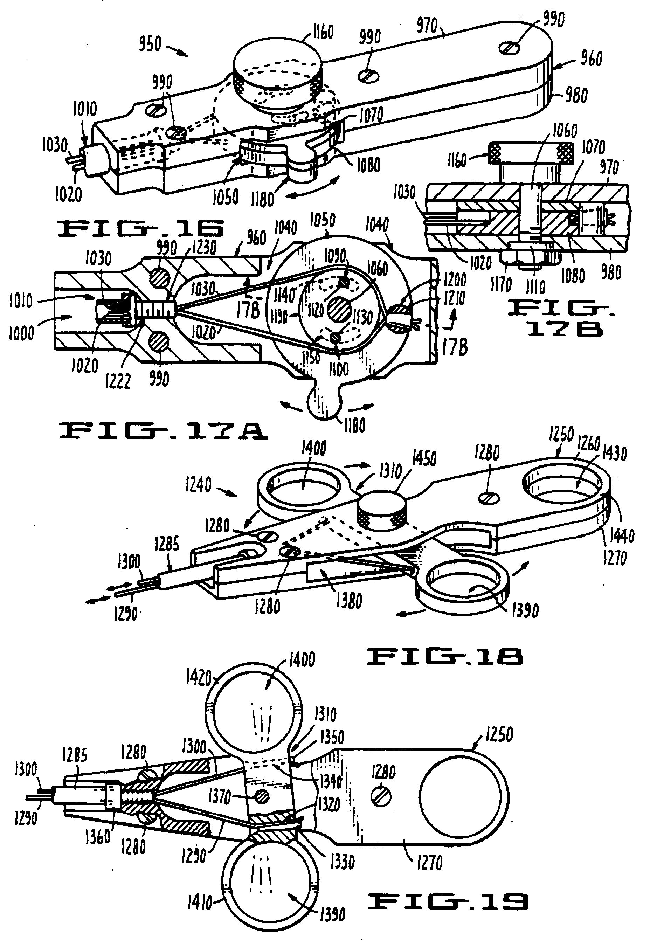

Figures 14 and 15 show a controller 790 of the invention which is an alternative to the controller 640 of the embodiment of the invention shown in Figures 11-13. The controller 790 forms a part of a steering mechanism having a distal end which may be identical to the embodiment of Figures 7 and 8, or may, as described below, be like the embodiment shown in Figures 21 and 22.

The controller 790 includes a housing 800 which receives a steering shaft 810, and also receives steering wires 820 and 830 through a wire retainer 835.

The housing 800 includes flanges 840 and 850 with coaxial bores (such as bore 860) therethrough, with bore 860 being formed through the flange 840 and another bore (not separately shown) being formed through flange 850. The bores have an axis which is substantially at right angles to a longitudinal axis of the housing 800 and the steering shaft 810. The controller 790 includes a handle 870 having forwardly disposed flanges 880 and 890 which include transverse bores therethrough (not separately shown) which are coaxial with the bores through the flanges 840 and 850. Extending through the bores in the flanges 840, 850, 880 and 890 is a shaft 900 (shown in Figure 15). The shaft includes an annular bearing 910. As shown in Figure 15, the steering wires 820 and 830 are disposed on opposite sides of the bearing 910 and are affixed to the handle 870 by means of screws 920 and 930. A tightening knob 940 (shown in Figure 14) is coupled to the shaft 900, preferably by threading onto an end thereof, and is used to adjust friction in the same manner as the knob 570 shown in Figure 9.

When the handle 870 is pulled to the left, as shown in dotted fashion in the lower portion of Figure 15, rearward tension is placed on the steering wire 830 and a forward force is exerted on the steering wire 820. This causes the distal tip of the steering shaft to bend to the right, as shown in dotted fashion on the upper side of the steering shaft 320 in Figure 7. Conversely, when the handle 870 is pushed to the right with respect to the housing 840, as shown in dotted fashion in the upper portion of Figure 15, rearward tension is placed on the steering wire 820 and a forward force is exerted on the steering wire 830, causing the distal tip of the steering shaft to bend to the left, as shown in dotted fashion on the lower side of the steering shaft 320 in Figure 7. As with the embodiment of Figure 9, the knob 570 may be tightened such that the handle 870 will maintain whatever position it is placed in, or it may be loosened to allow much freer play to the handle 870.

Figures 16, 17A and 17B show another embodiment of the controller of the invention. The controller 950 includes a housing 960 having an upper half 970 and a lower half 980. These halves are held together by screws 990. The housing 960 includes a bore 1000 in its distal end, for receiving a proximal end of a steering shaft 1010, in which steering wires 1020 and 1030 are positioned. The portion of the steering shaft 1010 and steering wires 1020 and 1030 to the left of Figure 16 may be identical to the distal portion of the steering shaft shown in Figures 7 and 8.

The housing 960 includes a cavity 1040 defined between the two halves 970 and 980, in which a disk 1050 is rotatably mounted, with its axis of rotation defined by a shaft 1060 disposed substantially at right angles to a longitudinal axis of the housing 960. The disk 1050 includes an upper half 1070 and a lower half 1080, into which are inserted dowels or pins 1090 and 1100. The pins 1090 and 1100 are received by coaxial bores (not separately shown) in the upper and lower halves 1070 and 1080, respectively. The pins 1090 and 1100 include heads or tips 1120 and 1130, which protrude above the upper half 1070 of the disk 1050. Corresponding arcuate slots 1140 and 1150 are provided in the lower surface of the upper half 970 of the housing 960, thus limiting the rotational travel of the disk 1050.

The steering wires 1020 and 1030 are clamped between the upper half 1070 and the lower half 1080. Attached to the upper end of the shaft 1060 is a knurled knob 1160, onto the lower end of which a nut 1170 is threaded, as shown in Figure 17B. The nut 1170 may include a press-nut portion 1110, which has a noncircular cross-section and is press-fit into the lower half 980 of the housing 960, as shown in Figure 17B. This allows the nut 1170 to be tightened without the use of a wrench.

The knob 1160 is used to adjust the friction imparted by the housing 960 to the disk 1050, in similar fashion to the knob 940 of the controller 790 shown in Figure 14.

A tab 1180 is formed on a side of the disk 1050, by protruding portions of the upper and lower halves 1070 and 1080. The tab 1180 forms a grip for the thumb of the operator. An additional tab (not separately shown) may be formed diametrically opposite the tab 1180.

The extreme proximal ends of the steering wires 1020 and 1030 are clamped in place around a bearing 1190 by means of a standard clamp 1200, which may include a conventional taper pin (like the taper pin 485 shown in Figure 10). The clamp 1200 is tightly received in a flared indentation 1210 formed in one or both of the halves 970 and 980 of the disk 960.

Thus, as the tab 1180 is pushed clockwise from the point of view of Figure 17A, rearward tension is placed on the steering wire 1030, and a forward force is exerted on the steering wire 1020. The distal tip of the steering shaft 1010 is thus turned to the right, as described previously with respect to the steering shaft 320 shown in Figure 7. Pushing on the tab 1180 counter-clockwise from the point of view of Figure 17A likewise turns the distal tip of the steering shaft 1010 to the left.

A tightening screw 1220 is preferably threaded into a bore 1230 in the housing 960, by which the tension on the steering wires 1020 and 1030 may be adjusted by screwing the tightening screw 1220 into or out of the bore 1230. This operates in the same fashion as the threaded bolt 510 shown in Figures 9 and 10.

Another embodiment of the invention is shown in Figures 18 and 19, depicting a controller 1240 which is an alternative to the controllers described above. The controller 1240 includes a housing 1250, including an upper half 1260 and a lower half 1270, which are held together by means of screws 1280. The housing receives a steering shaft 1285, in which are disposed steering wires 1290 and 1300. The distal portion of the steering shaft 1285 may be identical to the distal portion of the shaft 320 shown in Figure 7.

The steering wires 1290 and 1300 are attached to a rotator 1310, as shown in Figure 19. Any conventional attachment method may be used, but in the embodiment shown the extreme proximal end of the steering wire 1290 passes through a tapered slot 1320 formed in the rotator 1310, and a wedge or taper pin 1330 is tightly fitted into the slot 1320, thus preventing the steering wire 1290 from slipping out of the slot. Likewise, the steering wire 1300 is positioned in a slot 1340 and is maintained in position by a wedge or taper pin 1350. Tension on the steering wires 1290 and 1300 may be adjusted by means of a bolt 1360, just as with the tightening screw 1220 shown in Figure 17A.

The rotator 1310 is rotatably mounted on a shaft 1370 which is substantially at right angles to a longitudinal axis of the housing 1250 and the longitudinal axis of the steering shaft 1285. A slot 1380 is formed between the upper and lower housing halves 1260 and 1270, as shown in Figure 18. Thus, the operator of the mechanism may turn the distal end of the steering shaft 1285 to the left or right by rotating the rotator 1310 counterclockwise or clockwise, respectively. Finger holes 1390 and 1400 are formed by circular portions 1410 and 1420, which are in the preferred embodiment integral with the rotator 1310. This provides ease of operation of the controller 1240. Likewise, a finger hole or thumb hole 1430 is formed by a circular portion 1440 formed in the housing 1250.

The rotator 1310 is operated in essentially the same fashion as the disk 1050 shown in Figure 16, but provides a different type of hand hold for the physician. A tightening knob 1450 is provided to adjust the friction on the rotator 1310, just as with the knurled knob 1160 of the embodiment of Figure 16.

Figures 20-24 show an alternative embodiment of the invention, including a steering mechanism 1460 including steering wires 1470 and 1480, which terminate in distal tips 1490 and 1500, respectively. The steering mechanism 1460 includes a housing 1510, and shafts 1520 and 1530 which are transversely, rotatably mounted.

Carried on the upper end of shaft 1520 is a disk 1560 having an arcuate groove 1570 around its circumference. The disk 1560 is attached in a non-rotatable fashion to the shaft 1520. Mounted in the housing 1510 is a stop 1580, for limiting the rotational motion of the disks 1560 and hence of the shaft 1520, with the limit of rotation being determined by the arc length of the groove 1570. The shaft 1520 is rotated by turning the disk 1560.

Similarly, a disk 1590 is rigidly attached to the shaft 1530, and includes an arcuate groove 1600 around its perimeter. Another stop 1610 is mounted in the housing 1510, to limit the rotational motion of the disk 1590 and hence of the shaft 1530.

Knobs 1540 and 1550 are threaded onto the shafts 1520 and 1530, as shown in Figure 24, and provide rotational friction to the disks 1560 and 1590, respectively, in the same manner as the knob 570 shown in the embodiment of Figure 7.

As shown in Figure 23, the steering wire 1470 is attached to the shaft 1520 by passing the extreme proximal end thereof through a bore 1620 formed in the shaft 1520, and by fixing the proximal tip so that the steering wire 1470 cannot slip relative to the bore 1620. Similarly, steering wire 1480 is attached to the shaft 1530 through a bore 1630.

Affixation of each of the steering wires to the respective shafts may be accomplished by any standard means, such as by wrapping the steering wire around itself, or by welds, adhesives, clamps or the like. In a preferred embodiment, the steering wires 1470 and 1480 are attached to the shafts 1520 and 1530, respectively, by means of conventional taper pins 1632 and 1634, in a friction fit to prevent the wires 1470 and 1480 from accidentally coming loose.

In the embodiment of Figures 20-24, the steering wire 1470 is attached to the extreme distal tip of a lead spring 1640 carried within a steering shaft 1650. The lead spring is positioned within a slot of a ferrule 1660. The configuration of the lead spring 1640, the ferrule 1660, and the attachment of the steering wire 1470 to the lead spring 1640 may be identical to the configuration of lead spring 350, ferrule 400, and steering wire 360 shown in Figure 8. The lead spring 1640 is held in place relative to the steering shaft 1650 by means of an adhesive 1670. The adhesive 1670 may include an electrically conductive material, if the steering shaft 1650 is to be utilized in an embodiment wherein an electrode is desired at the tip of the steering shaft.

In this embodiment, the steering wire 1480 is attached at its distal tip to the lead spring 1640 at a point somewhat removed from the distal tip thereof, as shown in Figures 21 and 22. With this configuration, rotation of the shaft 1520 so as to place tension on the steering wire 1470 will cause the extreme distal tip of the steering shaft 1650 to turn to the left, while placing tension on the steering wire 1480 (by rotating the shaft 1530) will cause the steering shaft 1650 to turn to the right at the point of attachment of the steering wire 1480 to the lead spring 1640. Such a configuration is shown in Figure 21.

This embodiment has several advantages. For instance, tortuous paths may be negotiated by the tip of the steering shaft 1650 by operation of the knobs 1560 and 1590 while the physician views on a fluoroscope. Also, if the catheter (such as catheter 40 shown in Figure 35) is to be positioned in a vessel which is complicated in shape, the configuration of Figure 21, or variations thereon, may be the only way to position the distal tip of the steering shaft 1650 without placing too much stress on internal body structures. Another advantage is that the extreme distal tip of the steering shaft 1650 may be placed in position within the patient, and tension may then be taken off of the steering wire 1470, so that the distal tip of the steering shaft 1650 is free to flex, limited only by the flexibility of the lead spring 1640. However, at the same time, a bend may be retained in the portion of the steering shaft 1650 adjacent the attachment of the steering wire 1480 to the lead spring 1640, which is of particular importance where the catheter must make a turn very near the final emplacement point of the distal tip thereof, such as in the human heart.

Because the knobs 1560 and 1590 are operable independently of one another, the embodiment of Figure 20 may take the hook-shaped configuration shown in Figures 20 and 21. However, the offset attachment of the steering wires 1470 and 1480 shown in Figure 21 may also be used with other embodiments of the invention herein, indeed wherever two steering wires are used, such as in the embodiments of Figures 7, 11, 13, 16, and 18. In the embodiments where the steering wires are not independently operable, the hook-shaped configuration of Figure 21 is not achieved. However, flexibility in the guiding of the distal tip of the steering shaft is in each case provided, by allowing bends in the distal tip of the steering shaft to be made at different points along its length, such as at its extreme distal tip and somewhat further back thereof. This provides an extra measure of flexibility over the design of Figure 1.

Shapes other than the hook shape of Figure 21 may be achieved with different locations of attachment of the steering wires to the lead spring. Also, more than two steering wires may be used, if desired, with the various embodiments described herein. For instance, it may be desirable to have two offset steering wires attached to one side of the lead spring and one steering wire attached to the other side of the lead spring, to accommodate a particular configuration of emplacement. Alternatively, it may be useful for particular applications to attach steering wires at different angles circumferentially around a lead spring (which may be oval or polygonal in cross section). Other variations on the number, attachment and positioning of the steering wires may be made, and the shapes of the steering wires and the lead spring may be adapted accordingly.

Further flexibility may be accomplished by the present invention utilizing the configurations of Figures 33 and 34. In this embodiment, a steering shaft 1680 includes a distal tip 1690, having regions 1700 and 1710 of enlarged diameter. A lead spring 1720 is positioned within the distal tip 1690, and has its proximal end mounted in a ferrule 1730. A steering wire 1740 is attached at its extreme distal tip to the lead spring 1720, such as at weld 1750. The configuration of the lead spring 1720, ferrule 1730, and steering wire 1740 may be essentially identical to the embodiment of Figure 2.

When tension is placed on the steering wire 1730, it will be seen from Figure 34 that the distal end 1690 of the steering shaft 1680 takes sharp bends in two places, namely in the regions 1700 and 1710. This allows the physician greater flexibility in maneuvering around particular obstructions and negotiating difficult pathways through a vessel in the patient's body. Both this embodiment and the embodiment of Figures 20-24 may be carefully constructed such that the distance between bends and the resultant radii of curvature are adapted for a particular medical or other applications. For instance, the embodiments of Figures 20-24 or 33-34 may be custom designed for emplacement within the left ventricle of the heart, given the known shape of the heart. Other adaptations are easily accomplished with these designs.

## Claims

1. A steering mechanism, including:
a steering shaft (30; 320; 630; 810; 1010; 1285; 1680) having a proximal end (40; 330) and a distal end (160; 340; 1690) and a lumen therethrough;
a coiled spring (170) mounted within said steering shaft;
a lead spring (230; 350; 1640) positioned within said distal end;
at least one steering wire (120; 360, 370; 610, 620; 820, 830; 1020, 1030; 1290, 1300; 1470, 1480) extending through said lumen and having a distal end (150) attached to a first portion of said lead spring, for bending the lead spring towards said steering wire when tension is applied to the steering wire, thus bending the distal end of the steering shaft; and
a controller (15; 410; 640; 790; 950; 1240; 1460) positioned at the proximal end (40; 330) of the steering shaft (30; 320; 630; 810; 1010; 1285; 1680) and attached to the proximal end of said at least one steering wire, for placing tension on said steering wire;
characterised in that the lead spring (230; 350; 1640) is secured at the distal end of said coiled spring (170) against longitudinal movement relative to said distal end, said first portion of said lead spring extending beyond said coiled spring.

2. A steering mechanism according to claim 1, wherein the or each said steering wire extends through said coiled spring.

3. A steering mechanism according to claim 1 or claim 2, wherein said lead spring is secured by securing means including a ferrule (190; 400; 1660; 1730) mounted at the distal end of said coiled spring, said ferrule including a transverse slot (210, 220), and wherein said lead spring is mounted within said slot.

4. A steering mechanism according to claim 3, wherein said ferrule has a longitudinal bore, and the or each said steering wire extends through said longitudinal bore.

5. A steering mechanism according to claim 4, wherein each of said steering shaft and said longitudinal bore have an inner diameter which is less than the maximum thickness (265) of said lead spring and steering wire or wires.

6. A steering mechanism according to any preceding claim wherein the controller (410; 950; 1240; 1460) includes:
a housing (420; 960; 1250; 1510) attached to the steering shaft;
a shaft (450; 900; 1060; 1370; 1520) rotatably mounted in the housing, with the proximal end (470, 480) of at least one said steering wire attached to the shaft;
means (540; 870; 1180; 1390; 1560) attached to the shaft for rotating the shaft, for placing tension on the steering wire.

7. A steering mechanism according to claim 6 wherein said controller includes:
a second shaft (1530) rotatably mounted in the housing, with the proximal end of another said steering wire attached to the second shaft;
and means (1590) attached to the second shaft for rotating the second shaft, for placing tension on said another steering wire.

8. A steering mechanism according to claim 6 or claim 7 wherein said means attached to said shaft or shafts comprises a manually operable knob or handle (540; 870; 1180; 1390; 1560, 1590).

9. A steering mechanism according to any one of claims 6 to 8, including means (570, 580, 590, 600; 940; 1160; 1450; 1540, 1550) for adjusting rotational friction on said shaft or shafts.

10. A steering mechanism according to any one of claims 1 to 5 wherein the controller (15; 640) includes:
a housing having a first part (50, 60, 80) which is attached to the steering shaft and includes a transverse opening (680);
said housing having at least one second part (20; 720) which is longitudinally displaceable relative to said first part, said proximal end of a respective said steering wire (120; 610) being attached to said second part(s);
a first wedge (270; 650) slidably mounted in said transverse opening, and having a slot (300; 690) through which said second end of one said steering wire passes;
sliding of said first wedge serving to displace said second part relative to said first part to apply tension to said one steering wire.

11. A steering mechanism according to claim 10 wherein said housing comprises another second part (730) which is longitudinally displaceable relative to said first part, and comprising:
another said steering wire (620) having its proximal end attached to said another second part;
a second wedge (660) slidably mounted in said transverse opening, and having a slot (700) through which said second end of said another steering wire passes;
sliding of said second wedge serving to displace said another second part relative to said first part to apply tension to said another steering wire, the action of said second wedge being opposed to that of the first wedge.

12. A steering mechanism according to any preceding claim, including at least two said steering wires (1470, 1480) having distal ends attached to said lead spring at respective first locations which are longitudinally displaced along the lead spring.

13. A steering mechanism according to any preceding claim, including at least two said steering wires (1470, 1480) having distal ends attached to said lead spring at respective first locations which are on opposed sides of said lead spring.

14. A steering shaft according to any preceding claim wherein said steering shaft (1680) includes at least one section (1700, 1710) of enlarged diameter whereat said shaft will bend to a greater degree than the unenlarged section(s) of the shaft.

15. A steering mechanism according to any preceding claim wherein nothing is interposed laterally between said first portion of said lead spring and the distal end of said steering shaft, other than said steering wire or wires.

## Patentansprüche

1. Lenkmechanismus, welcher aufweist:
einen Lenkschaft (30; 320; 630; 810; 1010; 1285; 1680) mit einem proximalen Ende (40; 330) und einem distalen Ende (160; 340; 1690) sowie einem hierdurch verlaufenden Lumen bzw. Hohlraum;
eine innerhalb des Lenkschaftes angebrachte Schraubenfeder (170);
eine innerhalb des distalen Endes angeordnete Führungsfeder (230; 350; 1640);
mindestens einen Lenkdraht (120; 360, 370; 610, 620; 820, 830; 1020, 1030; 1290, 1300; 1470, 1480), welcher sich durch den Hohlraum erstreckt und ein an einem ersten Abschnitt der Führungsfeder angebrachtes distales Ende (150) aufweist, um die Führungsfeder beim Aufbringen einer Spannung auf den Lenkdraht zum Lenkdraht hin zu biegen, wodurch das distale Ende des Lenkschaftes ausgelenkt wird; und
eine am proximalen Ende (40; 330) des Lenkschaftes (30; 320; 630; 810; 1010; 1285; 1680) angeordnete und am proximalen Ende des mindestens einen Lenkdrahtes angebrachte Steuereinrichtung (15; 410; 640; 790; 950; 1240; 1460) zum Aufbringen einer Spannung auf den Lenkdraht;
dadurch gekennzeichnet, daß die Führungsfeder (230; 350; 1640) am distalen Ende der Schraubenfeder (170) gegen eine Bewegung in Längsrichtung relativ zu dem distalen Ende gesichert ist, wobei sich der erste Abschnitt der Führungsfeder über die Schraubenfeder hinaus erstreckt.

2. Lenkmechanismus nach Anspruch 1, dadurch gekennzeichnet, daß sich jeder der Lenkdrähte durch die Schraubenfeder hindurch erstreckt.

3. Lenkmechanismus nach Anspruch 1 oder 2, dadurch gekennzeichnet, daß die Führungsfeder mittels Sicherungseinrichtungen gesichert ist, welche einen am distalen Ende der Schraubenfeder angebrachten Haltering (190; 400; 1660; 1730) aufweisen, wobei der Haltering einen Querschlitz (210, 220) aufweist und die Führungsfeder in dem Schlitz angebracht ist.

4. Lenkmechanismus nach Anspruch 3, dadurch gekennzeichnet, daß der Haltering eine Längsbohrung aufweist, und daß sich der Lenkdraht bzw. jeder der Lenkdrähte durch die Längsbohrung erstreckt.

5. Lenkmechanismus nach Anspruch 4, dadurch gekennzeichnet, daß sowohl der Lenkschaft als auch die Längsbohrung einen Innendurchmesser aufweisen, der geringer als die maximale Dicke (265) der Führungsfeder und des Lenkdrahtes bzw. der Lenkdrähte ist.

6. Lenkmechanismus nach einem der vorhergehenden Ansprüche, dadurch gekennzeichnet, daß die Steuereinrichtung (410; 950; 1240; 1460) aufweist:
ein am Lenkschaft angebrachtes Gehäuse (420; 960; 1250; 1510);
eine im Gehäuse drehend gelagerte Welle (450; 900; 1060; 1370; 1520), wobei das proximale Ende (470, 480) wenigstens eines der Lenkdrähte an der Welle angebracht ist;
eine an der Welle angebrachte Einrichtung (540; 870; 1180; 1390; 1560) zum Drehen der Welle, um Spannung auf den Lenkdraht aufzubringen.

7. Lenkmechanismus nach Anspruch 6, dadurch gekennzeichnet, daß die Steuereinrichtung aufweist:
eine zweite drehbar im Gehäuse gelagerte Welle (1530), wobei das proximale Ende eines anderen Lenkdrahtes an der zweiten Welle angebracht ist;
und eine an der zweiten Welle angebrachte Einrichtung (1590) zum Drehen der Welle, um Spannung auf den anderen Lenkdraht aufzubringen.

8. Lenkmechanismus nach Anspruch 6 oder 7, dadurch gekennzeichnet, daß die an der Welle bzw. den Wellen angebrachte Einrichtung einen von Hand betätigbaren Knopf bzw. Griff (540; 870; 1180; 1390; 1560, 1590) aufweist.

9. Lenkmechanismus nach einem der Ansprüche 6 bis 8, dadurch gekennzeichnet, daß er Einrichtungen (570, 580, 590, 600; 940; 1160; 1450; 1540, 1550) zum Einstellen des Drehreibungswiderstandes an der Welle bzw. den Wellen aufweist.

10. Lenkmechanismus nach einem der Ansprüche 1 bis 5, dadurch gekennzeichnet, daß die Steuereinrichtung (15; 640) aufweist:
ein Gehäuse mit einem am Lenkschaft angebrachten ersten Teil (50, 60, 80), welcher eine Queröffnung (680) aufweist,
wobei das Gehäuse zumindest einen zweiten Teil (20; 720) aufweist, welcher relativ zum ersten Teil in Längsrichtung verschiebbar ist und wobei das proximale Ende eines jeweiligen Lenkdrahtes (120; 610) an dem bzw. den zweiten Teil(en) angebracht ist;
einen ersten Keil (270; 650), welcher gleitverschieblich in der Queröffnung angebracht ist und einen Schlitz (300; 690) aufweist, durch welchen hindurch das zweite Ende eines Lenkdrahtes verläuft,
wobei eine Gleitverschiebung des ersten Keiles dazu dient, den zweiten Teil relativ zum ersten Teil zu verschieben, um eine Spannung auf den einen Lenkdraht aufzubringen.

11. Lenkmechanismus nach Anspruch 10, dadurch gekennzeichnet, daß das Gehäuse einen weiteren zweiten Teil (730) aufweist, welcher relativ zum ersten Teil in Längsrichtung verschiebbar ist und aufweist:
einen weiteren Lenkdraht (620), dessen proximales Ende an dem weiteren zweiten Teil angebracht ist;
einen zweiten Keil (660), welcher gleitverschieblich in der Queröffnung angebracht ist und einen Schlitz (700) aufweist, durch welchen hindurch das zweite Ende des weiteren Lenkdrahtes verläuft,
wobei eine Gleitverschiebung des zweiten Keiles dazu dient, den weiteren zweiten Teil relativ zum ersten Teil zu verschieben, um eine Spannung auf den weiteren Lenkdraht aufzubringen, und wobei die Aktion des zweiten Keiles entgegengesetzt zu derjenigen des ersten Keiles wirkt.

12. Lenkmechanismus nach einem der vorhergehenden Ansprüche, dadurch gekennzeichnet, daß er mindestens die zwei Lenkdrähte (1470, 1480) mit distalen Enden aufweist, welche an der Führungsfeder an jeweiligen ersten Stellen angebracht sind, die sich versetzt in Längsrichtung entlang der Führungsfeder befinden.

13. Lenkmechanismus nach einem der vorhergehenden Ansprüche, dadurch gekennzeichnet, daß er mindestens die zwei Lenkdrähte (1470, 1480) aufweist, deren distale Enden an der Führungsfeder an jeweiligen ersten Stellen angebracht sind, die sich auf einander gegenüberliegenden Seiten der Führungsfeder befinden.

14. Lenkmechanismus nach einem der vorhergehenden Ansprüche, dadurch gekennzeichnet, daß der Lenkschaft (1680) wenigstens einen Abschnitt (1700, 1710) mit einem größeren Durchmesser aufweist, an welchem sich der Schaft stärker biegt bzw. ausgelenkt wird als der bzw. die nicht verbreiterte(n) Abschnitt(e) des Schaftes.

15. Lenkmechanismus nach einem der vorhergehenden Ansprüche, dadurch gekennzeichnet, daß außer dem Lenkdraht bzw. den Lenkdrähten keine weiteren Bauelemente zwischen dem ersten Abschnitt der Führungsfeder und dem distalen Ende des Lenkschaftes angeordnet sind.

## Revendications

1. Mécanisme de guidage comprenant :
un arbre de guidage (30 ; 320 ; 630 ; 810 ; 1010 ; 1285 ; 1680) ayant une extrémité proximale (40 ; 330) et une extrémité distale (160 ; 340 ; 1690) et un passage au-travers de celles-ci ;
un ressort en spirale (170) monté à l'intérieur dudit arbre de guidage ;
un ressort en plomb (230 ; 350 ; 1640) positionné à l'intérieur de ladite extrémité distale ;
au-moins un fil de guidage (120 ; 360, 370 ; 610,620 ; 820, 830 ; 1020, 1030 ; 1290, 1300 ; 1470, 1480) s'étendant au-travers dudit passage et présentant une extrémité distale (150) fixée à une première portion dudit ressort en plomb, afin d'incurver le ressort en plomb vers ledit fil de guidage lorsqu'une tension est appliquée au fil de guidage, recourbant ainsi l'extrémité distale de l'arbre de guidage ; et,
un organe de commande (15 ; 410 ; 640 ; 790 ; 950 ; 1240 ; 1460) positionné sur l'extrémité proximale (40 ; 330) de l'arbre de guidage (30 ; 320 ; 630 . 810 ; 1010 ; 1285 ; 1680) et fixé à l'extrémité proximale dudit au-moins un fil de guidage, afin d'exercer une tension sur ledit fil de guidage ;
caractérisé en ce que le ressort en plomb (230 ; 350 ; 1640) est fixé à l'extrémité distale dudit ressort en spirale (170), à l'encontre d'un mouvement longitudinal par rapport à ladite extrémité distale, ladite première portion dudit ressort en plomb s'étendant au-delà dudit ressort en spirale.

2. Mécanisme de guidage selon la revendication 1 dans lequel le ou chaque fil de guidage s'étend au-travers dudit ressort en spirale.

3. Mécanisme de guidage selon la revendication 1 ou la revendication 2 dans lequel ledit ressort en plomb est fixé à l'aide de moyens de fixation comportant une virole (190 ; 400 ; 1660 ; 1730) montée à l'extrémité distale dudit ressort en spirale, ladite virole comportant une fente transversale (210,220) et dans lequel ledit ressort en plomb est monté dans ladite fente.

4. Mécanisme de guidage selon la revendication 3 dans lequel ladite virole possède un alésage longitudinal et le ou chaque fil de guidage s'étend au-travers dudit alésage longitudinal.

5. Mécanisme de guidage selon la revendication 4 dans lequel ledit arbre de guidage et ledit alésage longitudinal possède chacun un diamètre interne qui est inférieur à l'épaisseur maximale (265) dudit ressort en plomb et du ou des fils de guidage.

6. Mécanisme de guidage selon l'une quelconque des revendications précédentes dans lequel l'organe de commande (410 ; 950 ; 1240 ; 1460) comprend :
un logement (420 ; 960 ; 1250 ; 1510) fixé à l'arbre de guidage ;
un arbre (450 ; 900 ; 1060 ; 1370 ; 1520) monté à rotation dans le logement, l'extrémité proximale (470, 480) dudit au-moins un fil de guidage étant fixée à l'arbre ;
des moyens (540 ; 870 ; 1180 ; 1390 ; 1560) fixés à l'arbre pour son entraînement en rotation, afin d'exercer une tension sur le fil de guidage.

7. Mécanisme de guidage selon la revendication 6 dans lequel ledit organe de commande comprend :
un second arbre (1530) monté à rotation dans le logement, avec l'extrémité proximale dudit autre fil de commande fixée au second arbre ;
et des moyens (1590) fixés audit second arbre pour l'entraîner en rotation afin d'exercer une tension sur ledit autre fil de guidage.

8. Mécanisme de guidage selon la revendication 6 ou 7 dans lequel lesdits moyens fixés audit arbre ou auxdits arbres comprennent une poignée ou un bouton pouvant être actionné manuellement (540 ; 870 ; 1180 ; 1390 ; 1560, 1590).

9. Mécanisme de guidage selon l'une quelconque des revendications 6 à 8 comprenant des moyens (570, 580, 590, 600 ; 940 ; 1160 ; 1450 ; 1540, 1550) pour régler le frottement de la rotation sur ledit arbre ou sur lesdits arbres.

10. Mécanisme de guidage selon l'une quelconque des revendications 1 à 5 dans lequel l'organe de commande (15 ; 640) comprend :
un logement ayant une première partie (50,60,80) qui est fixée à l'arbre de guidage et qui comprend une ouverture transversale (680) ;
ledit logement comprenant au-moins une seconde partie (20 ; 720) qui est déplaçable longitudinalement par rapport à ladite première partie, ladite extrémité proximale d'un fil de guidage respectif (120 ; 610) étant fixée à la ou lesdites secondes parties ;
une première clavette (270 ; 650) montée à coulissement dans ladite ouverture transversale et présentant une fente (300 ; 690) au-travers de laquelle passe ladite seconde extrémité dudit fil de guidage ;
le coulissement de ladite première clavette servant à déplacer ladite seconde partie par rapport à ladite première partie afin d'appliquer une tension audit fil de guidage.

11. Mécanisme de guidage selon la revendication 10 dans lequel ledit logement comporte une autre seconde partie (730) qui est déplaçable longitudinalement par rapport à ladite première partie et qui comprend :
- un autre fil de guidage (620) dont l'extrémité proximale est fixée à ladite seconde autre partie ;
- une seconde clavette (660) montée à coulissement dans ladite ouverture transversale et présentant une fente (700) au-travers de laquelle passe ladite seconde extrémité de l'autre fil de guidage ;
- le coulissement de ladite seconde clavette servant à déplacer ladite autre seconde partie par rapport à ladite première partie afin d'appliquer une tension à l'autre fil de guidage, l'action de ladite seconde clavette étant opposée à celle de la première clavette.

12. Mécanisme de guidage selon l'une quelconque des revendications précédentes comprenant au-moins deux fils de guidage (1470, 1480) dont les extrémités distales sont fixées audit ressort en plomb en des premiers emplacements respectifs qui sont déplacés longitudinalement le long du ressort en plomb.

13. Mécanisme de guidage selon l'une quelconque des revendications précédentes comprenant au-moins deux fils de guidage (1470, 1480) dont les extrémités distales sont fixées audit ressort en plomb en des premiers emplacements respectifs qui sont disposés sur des côtés opposés dudit ressort en plomb.

14. Mécanisme de guidage selon l'une quelconque des revendications précédentes dans lequel ledit arbre de guidage (1680) comprend au moins une section (1700, 1710), de diamètre agrandi afin que ledit arbre puisse s'incurver selon un degré plus important que la ou les sections non agrandies de l'arbre.

15. Mécanisme de guidage selon l'une quelconque des revendications précédentes dans lequel rien d'autre que ledit fil de guidage ou lesdits fils de guidage, n'est interposé latéralement entre ladite première portion dudit ressort en plomb et l'extrémité distale dudit arbre de guidage.
